# EUROPÄISCHE PATENTSCHRIFT

(11) **EP 1 402 885 B1**
(45) Veröffentlichungstag und Bekanntmachung des Hinweises auf die Patenterteilung: **13.07.2005**
(21) Anmeldenummer: 03017809.9
(22) Anmeldetag: 05.08.2003
(51) Int. Cl.: A61K 31/04

(54) **Medizinische Artikel aus einer Latex-Alternative**
Medical articles consisting of an alternative material to latex
Articles médicaux à base d'un matériel alternatif au latex

(30) Priorität: 29.08.2002 DE 10239738
(43) Veröffentlichungstag der Anmeldung: 31.03.2004
(73) Patentinhaber: RAUMEDIC AG, 95233 Helmbrechts (DE)
(72) Erfinder: Kühlein, Georg, 95111 Rehau (DE)

(56) Entgegenhaltungen:
- EP-A- 0 086 512
- EP-A- 0 766 972
- DE-A- 2 622 502

## Beschreibung

Naturgummi, der üblicherweise aus dem Gummibaum (Hevea Brasiliensis) gewonnen wird, ist eine milchigweiße Dispersion, die in der Trockenmasse zu 91 - 95 % aus Cis-1,4-Polyisopren besteht. Der Rest setzt sich zusammen aus Salzen und Proteinen. Einige dieser natürlichen Proteine können beim Menschen als Allergene wirken und lokale und systemische Reaktionen auslösen.
Die Auswirkungen sind individuell verschieden, die klinischen Symptome können von der Hautrötung über heuschnupfenartige Zustände bis hin zum anaphylaktischen Schock reichen.
Es werden Soforttyp-Allergien und Spätallergien beobachtet.

Man unterscheidet bei Naturgummi zwischen Gummilatex und Festkautschuk.

Im ersteren Fall wird der Gummilatex direkt als Ausgangsprodukt genutzt und die Formteile, z. B. Gummihandschuhe, Spritzen, Absaugkatheter, Blasenkatheter oder Beatmungsmasken, werden in einem Tauchprozess mit anschließende Vulkanisation hergestellt.
Der Gummilatex enthält eine hohe Konzentration an den allergen wirkenden Proteinen.

Wird durch einen Aufbereitungsprozess die Festsubstanz aus dem Latex isoliert, nennt man dieses Material "Festkautschuk". Dieser Festkautschuk wird mit Füllstoffen und funktionellen Beimischungen geknetet oder gewalzt und schließlich in einer Kompressions- oder Injektionsform vulkanisiert.

Festkautschuk und die daraus hergestellten Teile enthalten in Folge des Aufbereitungsschrittes deutlich weniger Proteine als die Latex-Tauchartikel, wirken aber immer noch, wenn auch in eingeschränktem Umfang, allergen.

Typische Artikel aus Festkautschuk sind Nadelschutzelemente, Spülstopfen, Dichtelemente, Schlauchverbinder und pharmazeutische Verschlüsse.

Da ein erheblicher Prozentsatz der Bevölkerung gegen Naturkautschuk allergisch reagiert, stellte sich für die vorliegende Erfindung die Aufgabe, besonders für Infusionsbestecke und medizinische Einmalartikel, eine Materialalternative bereitzustellen, die für die jeweilige Anwendung uneingeschränkt geeignet ist, medizinisch unbedenklich ist, keinerlei allergische Reaktionen auslöst und möglichst einfach verarbeitbar ist.

Die bisher üblichen Latexalternativen basieren ausschließlich auf Synthesekautschuken, hier insbesondere Butylkautschuk, Ethylen-Propylen-Kautschuk, StyrolButadien-Kautschuk oder Silikonkautschuk.

Überraschenderweise gelang die Lösung der Aufgabe durch Entwicklung von Artikeln, die zusammengesetzt sind aus PVC, Amidwachs, Weichmacher und Synthesekautschuk.

Zusammensetzungen von PVC, Weichmacher und Nitrilkautschuk werden insbesondere für folgende Anwendungen eingesetzt:
- Für Dichtungen im Fensterbereich, wo es auf eine hohe mechanische Belastbarkeit und ein hohes Rückstellvermögen bei Temperaturen bis 80°C ankommt.
- Für Schläuche, bei denen eine geringe Kriechneigung unter mechanischer und/oder thermischer Last wesentlich ist.

Hierfür werden üblicherweise Rezepturen der Shore-Härte A 65 eingesetzt, wie sie in nachfolgender Tabelle - als Richtrezeptur - beschrieben sind:

| **Bestandteile** | **Menge** |
|---|---|
| PVC (K-Wert 70) | 100 T |
| Weichmacher (DEHP) | 80 T |
| Nitrilkautschuk | 60 T |

Die nachfolgende Beschreibung der Erfindung zeigt, dass es nicht naheliegend war, basierend auf diesem Stand der Technik eine Latexalternative für die oben genannten Infusionsbestecke und medizinischen Einmalartikel zu finden.

Für diese Infusionsbestecke und medizinischen Einmalartikel sind nämlich folgende Eigenschaften besonders wichtig:
- frei von Allergenen
- Shore-Härte nicht größer als A 55
- hohe Rückstellfähigkeit
- gutes Gleitverhalten
- eine eindringende Hohlnadel darf keine Durchstanzung verursachen, die Wiederabdichtung nach DIN 58361, Teil 1, Pkt. 7.1.7 nach dem Herausziehen der Hohlnadel muss erfüllt werden.

Diese Eigenschaften werden erfindungsgemäß erreicht durch eine Zusammensetzung, die in der Hauptsache umfasst:
- PVC einer geringen Shore-Härte, jedoch eines hohen K-Wertes
- Weichmacher aus der Gruppe der Phthalsäureester
- Synthesekautschuk, ausgewählt aus der Gruppe Acrylatkautschuk, Nitrilkautschuk oder thermoplastisches Polyurethan
- Amidwachs (Bisstearylethylendiamin)
wobei insbesondere die völlig ungewöhnliche Kombination eines hohen K-Wertes bei der Rezeptierung von Shore-Härten kleiner A 50 und die - weit über die übliche Dosierung hinausgehende - Zugabe von Amidwachs für die gewünschten Eigenschaften des Latexersatzes essentiell waren.

Je nach Teilegeometrie und Anforderungsprofil beträgt - bezogen auf 100 Teile PVC - die Menge an eingesetztem Weichmacher zwischen 30 und 80 Teilen, bevorzugt 50 Teile, die Menge an Synthesekautschuk zwischen 30 und 150 Teilen, bevorzugt 50 - 80 Teile, die Menge an Amidwachs zwischen 1 und 4 Teilen, bevorzugt 2 - 3 Teile.
Die Shore-Härte der Rezeptur liegt je nach Anwendung zwischen A 40 und A 55, der K-Wert des PVC zwischen 80 und 100.

Nachfolgend soll in einem Ausführungsheispiel die Erfindung im Detail erläutert werden:
In einem PVC-Schnellmischer, bestehend aus einer Heiz-/Kühlkombination, gibt man zu 100 Teilen PVC mit einem K-Wert von 100 120 Teile DEHP und 10 Teile Hitzestabilisator auf Basis ESO/Calcium-/Zink-Stearat. Diese Komponenten werden zunächst in der Heizstufe bei Temperaturen oberhalb 90°C gemischt, dann - nach Absorption des Weichmachers durch die pulverförmigen Anteile der Mischung - 100 Teile teilvernetzten Nitrilkautschuks, der frei von Antioxidantien ist, zugegeben und der Mischvorgang weitere 5 Minuten fortgesetzt.
Anschließend wird das so erhaltene Agglomerat auf einem Doppelschneckenextruder granuliert.
Aus diesem Granulat stellt man einen Schlauchverbinder für die Infusion her.
Dieser hat kein allergenes Potential, verfügt über eine Shorehärte von A 45 und setzt einer eindringenden Hohlnadel so wenig Widerstand entgegen, dass eine Durchstanzung nicht erfolgt, vielmehr anschließend die Dichtigkeitsanforderungen nach DIN 58361, Teil 1, Pkt. 7.1.7 erfüllt.

## Patentansprüche

1. Medizinische Arbeitsmittel, die überwiegend zusammengesetzt sind aus PVC, Weichmacher, Amidwachs und Synthesekautschuk.

2. Medizinische Arbeitsmittel nach Anspruch 1,
**dadurch gekennzeichnet,**
**dass** das PVC über einen K-Wert von 80 - 100, vorzugsweise 80, verfügt.

3. Medizinische Arbeitsmittel nach Anspruch 1, **dadurch gekennzeichnet, dass** der Weichmacher ausgewählt ist aus der Gruppe Phthalsäureester, Adipate, Citrate, Trimellitate oder Cyclohexandicarboxylate.

4. Medizinische Arbeitsmittel nach Anspruch 1, **dadurch gekennzeichnet, dass** der Synthesekautschuk ausgewählt ist aus der Gruppe thermoplastisches Polyurethan, Nitrilkautschuk oder Acrylkautschuk.

5. Medizinische Arbeitsmittel nach Anspruch 1 oder 4, **dadurch gekennzeichnet, dass** der Anteil Synthesekautschuk in der Zusammensetzung zwischen 30 und 150 Teile, bevorzugt 50 - 80 Teile, beträgt.

6. Medizinische Arbeitsmittel nach Anspruch 1, **dadurch gekennzeichnet, dass** der Anteil des Amidwachses in der Zusammensetzung 1 - 4 Teile, bevorzugt 2-3 Teile, beträgt.

7. Medizinische Arbeitsmittel nach Anspruch 1, **dadurch gekennzeichnet, dass** zusätzlich übliche Additive zur Erhöhung der Thermostabilität enthalten sind.

8. Medizinische Arbeitsmittel nach Anspruch 1, **dadurch gekennzeichnet, dass** deren Shore-Härte zwischen A 40 und A 55 liegt.

9. Verwendung medizinischer Arbeitsmittel nach Anspruch 1, als Schläuche, Dichtelemente und Formteile.

10. Medizinische Arbeitsmittel nach Anspruch 1, **dadurch gekennzeichnet, dass** die Zusammensetzung nach den Ansprüchen 2 - 8 definiert ist.

## Claims

1. Medical equipment consisting mainly of PVC, plasticiser, amide wax and synthetic latex.

2. Medical equipment according to Claim 1,
**characterised by**:
the PVC has a K-value of 80 - 100, preferably 80.

3. Medical equipment according to Claim 1, **characterised by**: the plasticiser is chosen from the group of phthalic acid esters, adipates, citrates, trimellitates and cyclohexane dicarboxylates.

4. Medical equipment according to Claim 1, **characterised by**: the synthetic latex is chosen from the group of thermoplastic polyurethane, nitrile latex and acrylic latex.

5. Medical equipment according to Claim 1 or 4, **characterised by**: the proportion of synthetic latex in the composition is between 30 and 150 preferably 50 - 80 parts.

6. Medical equipment according to Claim 1, **characterised by**: the proportion of amide wax in the composition is 1 - 4 parts, preferably 2 - 3 parts.

7. Medical equipment according to Claim 1, **characterised by**: common additives to increase the thermal stability are also contained.

8. Medical equipment according to Claim 1, **characterised by**: its Shore hardness is between A 40 and A 55

9. Employment of medical equipment according to Claim 1 as tubes, sealing elements and mouldings.

10. Medical equipment according to Claim 1, **characterised by**: the composition is defined according to Claims 2 - 8.

## Revendications

1. Moyens de travail médicaux qui sont, pour le plus grand nombre, composés de PVC, de plastifiant, de cire d'amide et de caoutchouc de synthèse.

2. Moyens de travail médicaux selon la revendication 1,
**caractérisés en ce que**
le PVC dispose d'une valeur K de 80 à 100, de préférence de 80.

3. Moyens de travail médicaux selon la revendication1, **caractérisés en ce que** le plastifiant est sélectionné dans le groupe des esters phtaliques, des adipates, des citrates, des trimellitates ou des dicarboxylates de cyclohexane.

4. Moyens de travail médicaux selon la revendication 1, **caractérisés en ce que** le caoutchouc de synthèse est sélectionné dans le groupe du polyurétanne thermoplastique, du caoutchouc nitrile ou du caoutchouc acrylique.

5. Moyens de travail médicaux selon la revendication 1 ou 4, **caractérisés en ce que** la part de caoutchouc de synthèse dans la composition est de 30 à 150 parts, de préférence de 50 à 80 parts.

6. Moyens de travail médicaux selon la revendication 1, **caractérisés en ce que** la part de la cire d'amide dans la composition est de 1 à 4 parts, de préférence de 2 à 3 parts.

7. Moyens de travail médicaux selon la revendication 1, **caractérisés en ce qu'**en plus des additifs usuels destinés à accroître la stabilité thermique sont contenus

8. Moyens de travail médicaux selon la revendication 1, **caractérisés en ce que** leur dureté shore se situe entre A 40 et A 55

9. Utilisation de moyens de travail médicaux selon la revendication 1, sous forme de flexibles, d'éléments d'étanchéité et de pièces moulées.

10. Moyens de travail médicaux selon la revendication 1, **caractérisés en ce que** la composition est définie selon les revendications 2 à 8.
